# EUROPÄISCHE PATENTANMELDUNG

(11) **EP 1 536 235 A2**
(43) Veröffentlichungstag der Anmeldung: **01.06.2005**
(21) Anmeldenummer: 04029992.7
(22) Anmeldetag: 10.03.1999
(51) Int. Cl.: G01N 33/68

(54) **Verfahren zum Nachweis von Umweltverschmutzung unter Verwendung von 14-3-3 Proteinen als Biomarker.**

(30) Priorität: 12.03.1998 DE 19810721
(62) Teilanmeldung aus: 99908954.3
(71) Anmelder: MÜLLER, Werner E. G., 65203 Wiesbaden (DE); Schröder, Heinz C., 65189 Wiesbaden (DE)
(72) Erfinder: MÜLLER, Werner E. G., 65203 Wiesbaden (DE); Schröder, Heinz C., 65189 Wiesbaden (DE)
(74) Vertreter: Krauss, Jan B., Dr.

(57) **Zusammenfassung**

Gegenstand der Erfindung ist (1) die Anwendung des neuen Biomarkers 14-3-3-Protein(e) zur Detektion der Belastung von Süßgewässern, Meerwasser, Trinkwasser oder Bodenproben mit natürlichen und anthropogenen Umweltchemikalien, insbesondere polychlorierten Biphenylen (PCBs) und/oder Östrogenen/Xenoöstrogenen [(Xeno)Östrogenen] sowie (2) ein neues Verfahren (ELISA-Methode) zur schnellen qualitativen und quantitativen Messung der 14-3-3 Proteine. Das ELISA-Verfahren kann auch zum Nachweis des Auftretens von 14-3-3 Protein in Körperflüssigkeiten von Menschen und Tieren, die mit dem Erreger von Prion-Erkrankungen infiziert sind, benutzt werden.

## Beschreibung

Die vorliegende Erfindung betrifft (1) die Anwendung des neuen Biomarkers 14-3-3 Protein(e) zur Detektion der Belastung von Süßgewässern, Meerwasser, Trinkwasser oder Bodenproben mit natürlichen oder anthropogenen Umweltchemikalien, insbesondere polychlorierten Biphenylen (PCBs) und/oder Östrogenen/Xenoöstrogenen [(Xeno)Östrogenen] sowie (2) die Entwicklung eines neuen Verfahrens (eine ELISA-Methode) zur schnellen qualitativen und quantitativen Messung der 14-3-3 Proteine. Das ELISA-Verfahren kann auch zum Nachweis des Auftretens von 14-3-3 Protein in Körperflüssigkeiten von Menschen und Tieren, die mit dem Erreger der Prion-Erkrankungen infiziert sind, benutzt werden.

### 1. Beschreibung der Methode

### 1. 1. Prinzip

### 1.1.1 Induktion der Expression von 14-3-3 nach Einfluß von polychlorierten Biphenylen (PCBs) und (Xeno)Östrogenen)

Von den Erfindern wurde gezeigt daß ein neuartiges "lenkendes" Chaperon, 14-3-3, bei niederen aquatischen Invertebraten nach Einfluß von polychlorierten Biphenylen (PCBs) exprimiert wird. Somit war ein neuer Biomarker für PCB - zumindest für aquatische Invertebraten - gefunden; die bisherigen Verfahren, PCBs bei Invertebraten nachzuweisen, sind unzulänglich. Weiterhin wurde gefunden, dass die Expression von 14-3-3 auch durch (Xeno)Östrogene induzierbar ist. Der Biomarker 14-3-3 wurde zum Zwecke eines Effektmonitorings von PCB und von (Xeno)Östrogenen mit der Süsswasser-Muschel *Corbicula fluminea,* der Nordsee-Kliesche *Limanda limanda* sowie dem Mittelmeer-Schwamm *Suberites domuncula* als Bioindikator eingesetzt.

### 1.1.2 ELISA-Verfahren zur Quantifizierung von 14-3-3

Unsere Untersuchungen zeigten, daß sich ein ELISA zur Quantifizierung von 14-3-3 mit Rohextrakten aus Tieren nicht empfindlich genug aufbauen läßt. Deshalb haben wir einen ELISA entwickelt, der ein Anreicherungsverfahren voranstellt. Es ist bekannt, daß 14-3-3 an die phosphorylierte Erkennungssequenz RS[Phosphorylierungsstelle]xSxP bindet (Muslin et at, Cell 84, 889-897, 1996). Dazu wurde die Erkennungssequenz RS[P]xSxP synthetisch mit einem Biotin-Terminus hergestellt. Diese wurde in Wells von ELISA-Platten, die mit Streptavidin beschichtet waren, gegeben. Nach Bindung von RS[P] x S x P über Biotin und Streptavidin wurden > 100 µl von Extrakten aus Tieren in die Wells gegeben. Nach Waschen wurden die RS[P]xSxP-14-3-3 Komplexe über markierte Antikörper quantifiziert. Als Markierung kann zum Beispiel die alkalische Phosphatase verwendet werden; als Substrat zum Nachweis dient dann 5-Brom-4-Chlor-3-Indolylphosphat/Nitroblau-Tetrazolium.

### 1.1.3 Anwendung

Das beschriebene Verfahren kann sowohl in Felduntersuchungen als auch in Laboruntersuchungen (Hälterungsexperimenten) zum Biomonitoring (z. B. zur Überwachung kommunaler und industrieller Klärwerkszu- und -abläufe, Flußwässer, Meerwasser) unter Benutzung geeigneter Bioindikatoren angewandt werden. Untersucht werden die Wässer in Hälterungsexperimenten nach Direktinkubationen. Bei potentiell schwachen Effekten, z. B. bei Flußwässern, können die zu untersuchenden Wässer auch aufkonzentriert werden.

Weiterhin kann die beschriebene ELISA-Methode zum Nachweis von 14-3-3 in Zellen (z. B. peripheren Blutlymphozyten) oder Geweben (z. B. Gehirn) von Menschen und Tieren, die mit dem Erreger von Prion-Erkrankungen infiziert sind, benutzt werden.

### 1.2. Grundlagen

### 1.2.1 Umweltmonitoring: Biomarker und Bioindikatoren

Intensive Untersuchungen im Rahmen des Umweltmonitorings beschäftigen sich mit der Detektion von Rückständen, speziell von halogenierten Kohlenwasserstoffen und Schwermetallen. Die Ergebnisse zeigen, daß eine große Lücke bezüglich der quantitativen Abschätzung des Aussetzungsrisikos gegenüber chemischen Agenzien in der Umwelt existiert. Das Expositionsrisiko kann nicht einfach dadurch quantifiziert werden, daß die Konzentration der Xenobiotika in Geweben bestimmt wird. Viele Umweltchemikalien zeichnen sich dadurch aus, daß sie entweder nicht in Geweben des ausgewählten Bioindikators akkumulieren oder schnell metabolisiert werden. Sogar für persistierende Xenobiotika gibt es eine chemische Bestimmungsmethode, die lediglich eine Momentaufnahme darstellt und häufig nur wenig Auskunft über das Muster der Exposition gibt, welches zur aktuellen Konzentration der Umweltchemikalie geführt hat. Weiterhin ist die Beziehung zwischen Gewebekonzentration und einer ggfs. toxischen Antwort komplex und meist noch unverstanden. Erschwerend kommt noch hinzu, daß Xenobiotika in der Umwelt als komplexe Mischungen vorliegen, die synergistische oder auch antagonistische Wechselwirkungen zeigen können.

Ein informativer Weg zur quantitativen Erfassung des Expositionsrisikos gegenüber (einer) Umweltchemikalie(n) und des daraus entstehenden potentiellen Einflusses auf den Organismus ist der Einsatz von Biomarkern bei ausgewählten Bioindikatoren.

### 1.2.2 Polychlorierte Biphenyle

Polychlorierte Biphenyle (PCBs) wurden seit den frühen 30-iger Jahren in elektrischen Geräten, bei der Herstellung von Farbstoffen, Plastikwaren, Schutzmaterialien sowie von Pestiziden, Feuerlöschmitteln und organischen Verdünnern benutzt. Sowohl diese weite Verbreitung als auch die nicht sachgerechte Entsorgung haben dazu geführt, daß PCBs ubiquitär in der Umwelt zu finden sind. Seit Mitte der 80-iger Jahre ist die Herstellung der PCBs in den USA und in Westeuropa verboten. Trotzdem sind PCBs auch heute noch in grossem Masse in unserer Umwelt vorhanden aufgrund ihrer hohen Bioakkumulation (Abramowicz, Environ. Health Perspect. 103, 97-99, 1995) und der geringen Biotransformation (Jedamski-Grymlas et al., Ecotoxicol. Environm. Saf. 31, 49-56, 1995). PCBs lassen sich in der Luft, im Boden, in Sedimenten, in marinen Gewässern und auch speziell in Süßgewässern nachweisen; sie sind in der Nahrungskette - Fisch, Fleisch von Wildtieren - und in menschlichem Fettgewebe aufzufinden (Voogt et al., Int. J. Environ. Anal. Chem. 40, 1-46, 1990).

Der umweltbiologisch bedeutsame und molekularbiologisch interessante Effekt einiger PCBs ist ihr stimulierender Einfluß auf den Aryl-Kohlenwasserstoff- Rezeptor (Ah-Rezeptor) (Ahlborg et al., Nord 1992, 26. Copenhagen: Nordic Council of Ministers, 1992), der als Liganden-induzierbarer Transkriptionsfaktor fungiert (Safe, Pharmacol. Ther. 67, 247-281, 1995). Daran binden neben PCBs auch andere Xenobiotika, wie Dioxine und Dibenzofurane, die auch als Kontaminationen der PCBs vorkommen. Unter den Proteinen, die durch PCBs induziert werden, sind besonders Stelle die Cytochrom P-450 Proteine (CYP) zu nennen (Shane et al., Mut. Res. 377, 1-11, 1997). Deshalb wurde die Induzierbarkeit der CYP-Gene als Maß für eine Belastung an PCBs herangezogen; z. B. wurde die CYP1A-Expression in enzymatischen Ansätzen (Dehnen et al., Anal. Biochem. 53, 373-383, 1973), über immunologische Detektionsverfahren (Williams und Buhler, Biochem. Pharmacol. 33, 3742-3753, 1984), und/oder durch Quantifizierung der entsprechenden mRNAs (Heilman et al., DNA 7, 379-387, 1988) bestimmt. Es ist etabliert, dass das Mass der CYP1A-Induzierbarkeit eine relativ verlässliche Aussage über eine PCB-Belastung bei Fischen zulässt (Stegeman et al., in: Huggett et al. (eds), Biomarkers: biochemical, physiological and histological markers of anthropogenic stress. Boca Raton, FL: Lewis, S. 235-335, 1992).

Jedoch gelten die CYP-verwandten Enzyme bei Invertebraten, wie bei Mytilus edulis, nicht als zuverlässige Biomarker für PCBs, da in diesen Organismen sowohl die entsprechenden Proteine als auch deren kodierende Gene kaum untersucht sind (Livingstone, Mar. Ecol. Prog. Ser. 46, 91-100, 1988). Deshalb ist die Entwicklung neuer Biomarker für PCBs bei Invertebraten angezeigt.

PCBs werden aufgrund ihrer chemischen Struktur und (chemischen) Eigenschaften in verschiedene Klassen, wie non-ortho- und mono-ortho-substituierte PCBs eingeteilt. Funktionell lassen sich die PCBs in toxische und in hormonähnliche Substanzen unterscheiden (Toppari et al., Environ. Health Perspect. 104 [Suppl. 4], 741-803, 1996). Toxizitätstests sind natürlich für PCBs bei Vertebraten wie auch bei Invertebraten vorhanden. Jedoch fehlen adäquate Testsysteme zum Nachweis einer subtoxischen Wirkung von PCBs bei Invertebraten im allgemeinen und einer (Xeno)Östrogenenlanti(Xeno)Östrogenen Wirkung von PCBs im besonderen bei Invertebraten.

### 1.2.3 (Xeno)Östrogene

(Xeno)Östrogene stellen Xenobiotika dar, die einen (Xeno)Östrogenen oder einen anti(Xeno)Östrogenen Effekt bei Metazoen auslösen. Hierunter nehmen die PCBs eine herausragende Stellung ein. Es ist bekannt, daß nieder-chlorierte PCBs, wie Aroclor 1221, 1232, 1242 oder 1248 (Xeno)Östrogene/anti(Xeno)Östrogene Wirkung zeigen, während höherchlorierte PCBs toxischer wirken (Toppari et al., Environ. Health Perspect. 104 [Suppl. 4], 741-803, 1996). Bei Vertebraten steht mit Vitellogenin ein verläßlicher Biomarker zur Verfügung. Mittels Antikörper oder Gensonden läßt sich die Expression von Vitellogenin bei Vertebraten in Serum und Gewebe männlicher Tiere quantifizieren (Heppell et al., Environ. Heath Persp. 103 [Suppl. 7], 9-15, 1995). Wir haben nach analogen Biomarkern bei Invertebraten gesucht; mit der Klonierung des Genes, das für das Protein 14-3-3 kodiert, waren wir erfolgreich.

### 1.2.4 14-3-3, ein neuartiges Chaperon

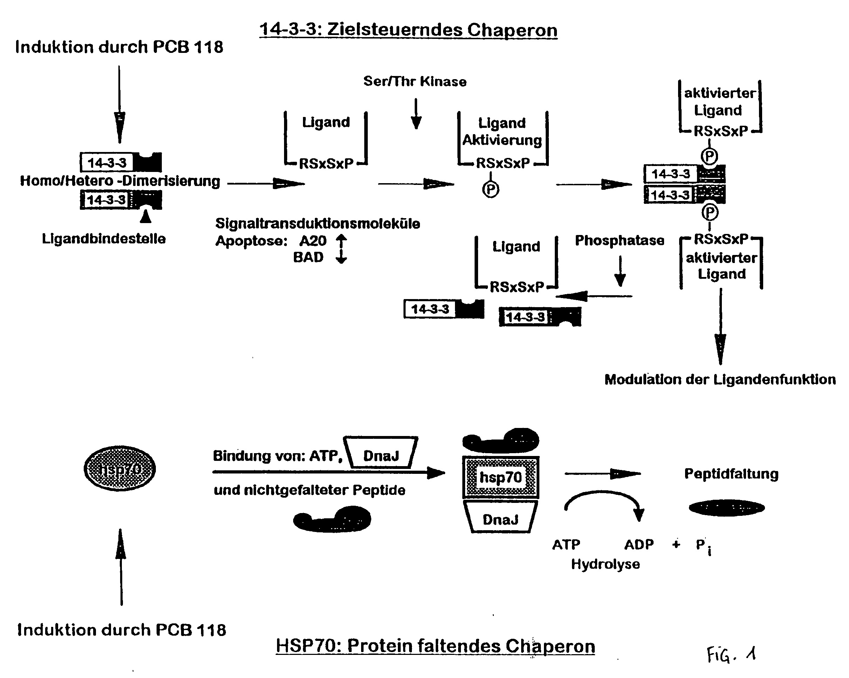

Das Hitzeschockprotein HSP70 wurde als ein Biomarker etabliert, der zur Indikation von Umweltstreß, speziell von osmotischem Streß und Belastung durch Schwermetalle, dient (Koziol et al., Canad. Technical Report of Fisheries and Aquatic Sci. 2093, 104-109, 1996; Krasko et al., Aquatic Toxicol. 37, 157-168, 1997; Koziol et al., Marine Ecol.; Progr. Ser. 154, 261-268, 1997). HSP70 stellt ein molekulares Chaperon dar, dessen Hauptfunktion es ist, die funktionsgerechte Faltung eines Proteins zu kontrollieren und zu erhalten (Becker und Craig, Eur. J. Biochem. 219, 11-23, 1994); ABB. 1. Im Gegensatz dazu bindet das Protein 14-3-3 (Aitken et al., Trends Biochem. Sci. 17, 498-501, 1992) an Funktionsmoleküle, wie (i) an Rezeptoren (z. B. dem Adrenodixon Präkursor) (Alam et al., J. Biochem. 116, 416-425,1994), (ii) an Signaltransduktions-Proteine wie Raf-1 (Muslin et al., Cell 84, 889-897,1996) oder (iii) an Schlüsselmoleküle der Apoptose (wie das BAD-Molekül oder A20) (Zha et al., Cell 87, 619-628, 1996) und verhindert den Transport dieser Moleküle an ihren Funktionsort; ABB. 1. Somit stellt 14-3-3 ein neuartiges Chaperon dar, dessen Funktion es ist, an andere Proteine zu binden und diese in bestimmten Zellkompartimenten zu "fixieren". 14-3-3 kontrolliert essentielle Funktionen der Zelle und "immobilisiert" deren Metabolismus (Aitken et al., Trends Biochem. Sci. 17, 498-501, 1992). Zunächst als neuronales Protein entdeckt, gilt 14-3-3 heute als ubiquitär vorkommendes Protein, das für die genannten Prozesse aller Metazoa von entscheidender Bedeutung ist; die Familie der 14-3-3 Proteine umfaßt sieben Isoformen (Aitken et al., Trends Biochem. Sci. 17, 498-501, 1992).

### 1.2.5 14-3-3: Biomarker für PCB

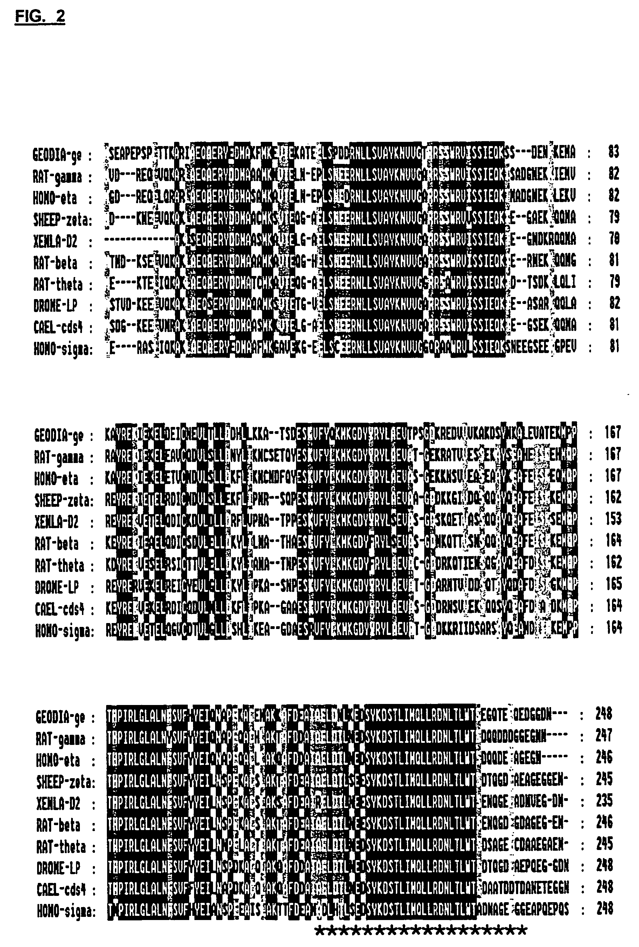

Den Antragstellern gelang es erstmalig zu zeigen, daß es sich bei 14-3-3 um durch PCB induzierbare Proteine - in den ersten Versuchen der Antragsteller nachgewiesen für marine Schwämme - handelt. Hierzu war zunächst die Klonierung von 14-3-3 nötig; ABB. 2. Anschliessend wurde die cDNA als Gensonde zum Nachweis der Expression auf der Ebene der mRNA und die entsprechenden Antikörper zur Bestimmung der Proteinmenge eingesetzt.

### 1.2.6 14-3-3: Biomarker für (Xeno)Östrogene

Wir konnten zeigen, daß PCB sowie 17 beta-Östradiol die Expression von 14-3-3 in niederen Invertebraten stark induziert. Weiter konnten wir nachweisen, daß nach Kombination beider Substanzen eine Potenzierung der Expression von 14-3-3 eintrat.

### 2. Beschreibung der Anwendung und des Verfahrens

### 2.1 Materialien

Die bei den nachfolgend beschriebenen Experimenten verwendeten Chemikalien wurden von folgenden Firmen bezogen: Restriktions-Endonucleasen und andere Enzyme für rekombinante DNA-Techniken und Vektoren von Stratagene (Heidelberg, Deutschland), QIAGEN (Hilden, Deutschland) und USB (Cleveland, OH, USA); Kornöl (C8267) from Sigma (Deisenhofen, Deutschland); TRIzol Reagenz von GibcoBRL (Grand Island, NY, USA); CDP [Dinatrium 2-Chlor-5-(4-methoxyspiro-1,2-dioxetane-3,2'-(5'-chlor)-tricyclo[3.3.1.13,7]decan-4-yl)phenylphosphat] von Boehringer Mannheim (Mannheim, Deutschland). Der polyklonale Antikörper (Kaninchen-IgG; Kat.-Nr. PC70) gegen die konservierten Aminosäurereste der 14-3-3 Proteinfamilie und die 14-3-3 Peptide aa221 bis aa242 wurden von Calbiochem/Oncogene (Cambridge, MA, USA) erhalten. PCB 118 wurde von der Dr. Ehrenstorfer GmbH (Augsburg, Deutschland) erworben.

### 2.2 Extraktgewinnung aus den Gewässern

Die Extrakte aus den Gewässern werden gegebenenfalls über Amberlite XAD-7 konzentriert. Die Fraktionen, die die organischen Halogene, PCBs, Phenole etc. enthalten, werden entsprechend früher publizierter Verfahren gewonnen (Müller et al., Environm. Toxicol. Chem. 14, 1203-1208, 1995).

### 2.3 Nachweis der Expression von 14-3-3 auf mRNA-Ebene

Aufgrund der Tatsache, daß weite Bereiche der verschiedenen 14-3-3 Isoformen sehr konserviert sind (ABB. 2), können degenerierte Primer zur Identifikation und zur Quantifizierung der mRNA eingesetzt werden. Für die quantitative PCR- Analyse wurde von den Erfindern bereits ein Primer, gerichtet gegen aa47-aa53; VAYKNVVG, 5'-GTKGCCTACAARAAYGTGGT-3' [K = G/T, R = NG und Y = C/T; Vorwärtsprimer] mit Erfolg angewandt.

Für eine Quantifizierung der mRNA für 14-3-3 mittels Northern-Blotting Verfahren werden Gensonden hergestellt, die von den konservierten Anfangsbereichen [aa45-aa57] bis zu den konservierten Endbereichen [aa214-aa234] des 14-3-3 Proteins reichen (ABB. 2). Als Methodik wird die PCR-Klonierung eingesetzt. Die erhaltenen APPROX 500 bp langen Sonden eignen sich erfahrungsgemäß gut für Northern-Blotting-Untersuchungen.

Die quantitative Auswertung der Signale kann zum Beispiel mit Hilfe des Chemilumineszenz-Verfahrens (Stanley und Kricka, Bioluminescence and chemiluminescence: current status. John Wiley & Sons, New York, 1990) in Verbindung mit einem Phospholmager (z. B. GS-525 Molecular Imager der Firma Bio-Rad) erfolgen; Dinatrium 2-Chlor-5-(4-methoxyspiro 1,2-dioxetane-3,2'-(5'-chlor)-tricyclo[3.3.1.13,7]decan-4-yl)phenyl-phosphat (CDP) wird hierfür als Substrat angewandt.

### 2.4 Nachweis der Expression von 14-3-3 auf Protein-Ebene: Western-Blotting

Der Nachweis der Expression von 14-3-3 auf Protein-Ebene erfolgt mittels Antikörper. Entweder können kommerzielle (z. B. von der Firma Calbiochem/Oncogene) oder gegen das rekombinante Invertebraten-Protein 14-3-3 aus *Geodia cydonium* gezogene Antikörper eingesetzt werden. Zur Quantifizierung der 14-3-3 Proteine werden Western-Blots mit Extrakten des Bioindikators hergestellt. Die Signale können wieder unter Anwendung des Chemilumineszenz-Verfahrens quantifiziert werden. Die Gewebeextrakte können z. B. durch Homogenisierung in einem Phosphat-Puffer, enthaltend 1 mM EDTA und 1 mM Phenylmethylsulfonylfluorid, erhalten werden.

### Beispiel 1 Anwendung von 14-3-3 als Biomarker für PCB: Nachweis mittels Northern- und Western-Blotting

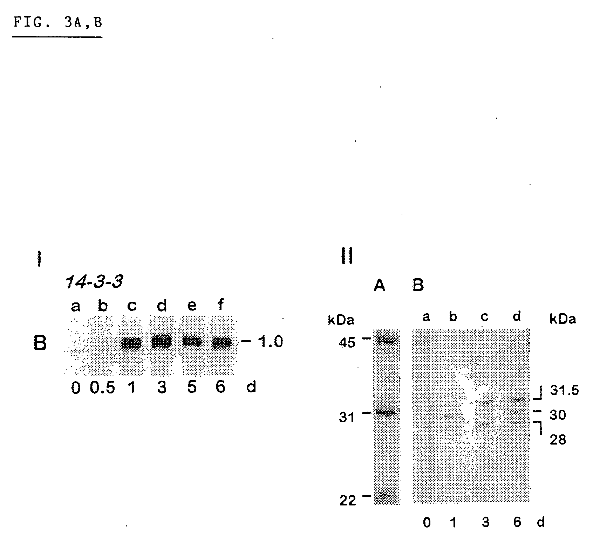

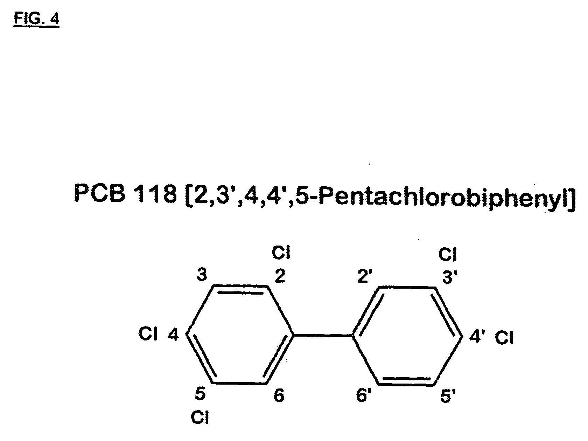

Die 14-3-3 cDNA des Meeresschwammes *Geodia cydonium* wurde als Gensonde zum Nachweis der Expression auf der Ebene der mRNA und die entsprechenden Antikörper zur Bestimmung der Proteinmenge eingesetzt; ABB. 3A und B. Bei den durchgeführten Hälterungsuntersuchungen wurde als Modell-PCB das PCB 118 verwendet (ABB. 4).

Durchfiihrung:
(a) Exposition von *G. cydonium* gegenüber PCB 118: 1 ml PCB 118 (0.1 mg/ml in Kornöl) wurde in 40 g Schwammgewebe injiziert. Die Proben wurden in Seewasser für bis zu 6 Tage in 20 1 Aquarien bei 17°C unter kontinuierlicher Belüftung inkubiert; das Wasser wurde einmal am Tag 2 gewechselt. Aliquote Teile des Gewebes (ungefähr je 200 mg) wurden zum Zeitpunkt Null oder nach einer Inkubationszeit von 0.5, 1, 3, 5 und 6 Tagen entnommen. Diese aliquoten Teile wurden sofort in flüssigem Stickstoff eingefroren und bei -80 DEG C aufbewahrt.
(b) Extraktion: Extrakte zur Bestimmung der Menge an 14-3-3 wurden durch Mörsern der gefrorenen Gewebeproben in dem dreifachen Volumen an Phosphat- Puffer unter Zusatz von 1 mM EDTA und 1 mM Phenylmethylsulfonylfluorid gewonnen. RNA wurde aus dem in flüssigem Stickstoff pulverisierten Schwammgewebe mit Hilfe des TRIzol Reagenz (GibcoBRL) nach den Angaben des Herstellers extrahiert.

Die Hälterungsuntersuchungen mit *G. cydonium* zeigten, daß PCB 118 die Expression des 14-3-3-Gens von nicht nachweisbar auf sehr hohe Werte induziert (ABB. 3A und B).

Vergleichbar starke und eindeutige Effekte konnten bisher noch mit keinem anderen Biomarker für PCB bei Invertebraten nachgewiesen werden.

Weiterhin ist bemerkenswert und wichtig für eine potentielle Anwendung als Biomarker bei anderen aquatischen Invertebraten, daß sowohl die Antikörper gegen 14-3-3 des Schwammes als auch die Gensonde von *G. cydonium* mit den homologen Proteinen/RNAs höherer Invertebraten und Vertebraten kreuzreagieren/hybridisiert.

### Beispiel 2 Anwendung von 14-3-3 als Biomarker für (Xeno)Östrogene: Nachweis mittels Northern-Blotting

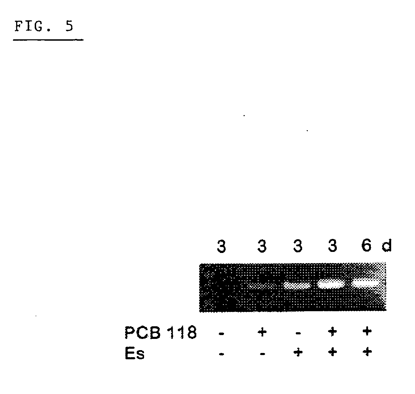

Durch Northern-Blotting konnte gezeigt werden, daß PCB sowie 17 beta-Östradiol die Expression von 14-3-3 in niederen Invertebraten stark induziert (ABB. 5). Weiter konnte nachgewiesen werden, daß nach Kombination beider Substanzen eine Potenzierung der Expression von 14-3-3 eintrat. In Abwesenheit beider Agenzien läßt sich 14-3-3 nicht detektieren. Während das Niveau der mRNA von 14-3-3 bei Applikation von PCB 118 bei 30% und von 17 beta-Östradiol bei 100% Bezugswert] lag, induzierten beide Chemikalien die Expression von 14-3-3 auf 550%. Die Induktion ist nach 3 Tagen abgeschlossen.

### 2.5 Nachweis der Expression von 14-3-3 mittels eines ELISA-Verfahrens

### 2.5.1 Präparation der ELISA-Mikrotiterplatten

14-3-3 ist ein spezifisch Phosphoserin-bindendes Protein; das 14-3-3 Bindemotiv ist identifiziert (Muslin et al., Cell 84, 889-897, 1996). In dem von uns entwickelten ELISA haben wir das Peptid LPKINRSApSEPSLHR (pS = Phosphoserin), an das 14-3-3 mit hoher Affinität bindet (Muslin et al., Cell 84, 889-897, 1996), N-terminal über zwei Alanine als Spacer mit einem Cystein verknüpft.

Variante 1: Hierbei wurde das chemisch synthetisierte Peptid CAALPKINRSApSEPSLHR durch Reaktion der Sulfhydrylgruppe des N-terminalen Cysteins an Reacti-Bind TM Maleimid-aktivierte Mikrotiterplatten (Kapazität: 100-150 pmol SH-enthaltende Peptide pro Well; Firma Pierce, Rockford, IL, USA) kovalent gebunden. Vorgegangen wurde hierbei nach den Instruktionen des Herstellers. Überschüssige Maleimidgruppen auf den Platten wurden durch einstündige Inkubation der Platten mit einer Cysteinlösung (10 µg/ml) blockiert.

Vor der Koppelung an die Platten wurde die Peptidlösung mit Ellman's Reagens auf die Gegenwart von SH-Gruppen getestet. Gegebenenfalls (im Falle einer Oxidation der SH-Gruppen unter Ausbildung von Disulfidbindungen) wurde das Peptid mit dem ReduceImm Kit von der Firma Pierce (siehe oben) behandelt.

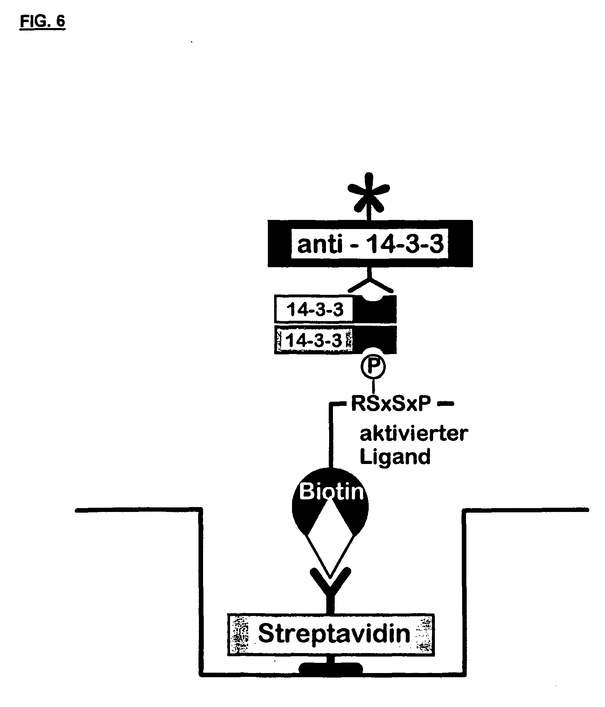

Variante 2: Bei diesem alternativen Verfahren wurde das chemisch synthetisierte Peptid CAALPKINRSApSEPSLHR durch Reaktion der Sulfhydrylgruppe des N-terminalen Cysteins an das EZ-Link TM Biotinylierungs-Reagens Biotin-BMCC 1-Biotinamido-4-[4'-(maleimidomethyl)-cyclohexanecarboxyamido]butane von der Firma Pierce (siehe oben) kovalent gebunden. Die Bindung der biotinylierten Proteine an die Mikrotiterplatten erfolgt in diesem Fall über Streptavidin (Verwendung von Streptavidin-beschichteten Mikrotiterplatten, ABB.6).

### 2.5.2 Durchführung des ELISA

Die Durchführung erfolgt in folgenden Schritten:
Variante 1
   1a. Inkubation der Streptavidin-beschichteten Mikrotiterplatten mit dem Biotingekoppelten Peptid.
   2a. Zugabe von 100 µl Extrakt aus den untersuchten Tiergeweben in die Wells nach Bindung des Peptids über Biotin und Streptavidin.
   Oder:
   1a. Inkubation der Extrakte aus den untersuchten Tiergeweben mit dem Biotingekoppelten Peptid.
   2a. Zugabe von 100 µl der Inkubationsmischungen nach Bindung des Biotin-Peptids an 14-3-3 in die Wells.
   3. Waschen.
   4. Quantifizierung der Peptid-14-3-3 Komplexe über markierte Antikörper. Als Markierung kann zum Beispiel die alkalische Phosphatase verwendet werden; als Substrat zum Nachweis dient dann 5-Brom-4-Chlor-3-Indolylphosphat/Nitroblau-Tetrazolium.
   5. Detektion mittels eines ELISA-Platten-Readers.
Variante 2
   1. Zugabe von 100 µl Extrakt in die Wells der Mikrotiterplatten mit dem gekoppelten Peptid und Inkubation.
   2. Waschen.
   3. Quantifizierung der Peptid-14-3-3 Komplexe über markierte Antikörper (siehe Variante 1).
   4. Detektion mittels eines ELISA-Platten-Readers.

### Beispiel 3: Quantifizierung von 14-3-3 in Klieschen nach Exposition gegenüber PCBs und Cadmium: Nachweis mittels des beschriebenen ELISA-Verfahrens

Die in Tabelle 1 angegebenen Mengen an PCB 77, PCB 118 und PCB 153 wurden nach Auflösung in Kornöl in Klieschen (*Limanda limanda*) injiziert. Die Injektion der angegebenen Mengen an Cadmium (Tabelle 2) erfolgte nach Lösung in PBS. Nach den angegebenen Zeiträumen wurde den Tieren die Leber entnommen und sofort eingefroren. Die Bestimmung des Gehalts an 14-3-3 in den Lebern erfolgte nach Homogenisation durch Mörsern der gefrorenen Gewebeproben in dem dreifachen Volumen an Phosphat-Puffer mit 1 mM EDTA und 1 mM Phenylmethylsulfonylfluorid.

**Tabelle 1:**

| Injektion [i. p.] von 0.03 mg PCB pro kg *Limanda limanda* (Kliesche- Fisch). Nach 5 Tagen wurden die Lebern entnommen und die Menge an 14-3-3 in dem angegebenen ELISA bestimmt. Die optischen Dichten sind in OD-Einheiten angegeben | |
|---|---|
| **Verbindung:** | **14-3-3 Protein (OD-Einheit)** |
| PCB77 | 0,97 |
| PCB 118 | 0,74 |
| PCB153 | 0,81 |
| Kontrolle | 0,12 |

**Tabelle 2:**

| Injektion [i. p.] von Cadmium in *Limanda limanda* (Kliesche-Fisch). Nach 5 Tagen wurden die Lebern entnommen und die Menge an 14-3-3 in dem angegebenen ELISA bestimmt. Die optischen Dichten sind in OD-Einheiten angegeben | |
|---|---|
| **Cadmium (mg/kg):** | **14-3-3 Protein (OD-Einheit)** |
| 0,01 | 0,30 |
| 0,03 | 0,75 |
| 0,1 | 0,98 |
| Kontrolle | 0,12 |

### Beispiel 4. Quantifizierung von 14-3-3 Protein in Rinderserum und Liquor cerebrospinalis : Nachweis mittels des ELISA-Verfahrens

Bei Prionen-Erkrankungen (BSE bei Rindern, Scrapie bei Schafen, Creutzfeldt Jakob-Erkrankung beim Menschen u. a.) kommt es zu einer Erhöhung der Konzentration an 14-3-3 Protein im Liquor cerebrospinalis (Cerebrospinalflüssigkeit) (Jones et al., Veterinary Record 139,360-363,1996; Lee and Harrington, Electrophoresis 18,502-506,1997 ; Lee and Harrington, Veterinary Record 140, 206-207,1997 ; Zerr et al., Ann. Neurol. 43,32-40,1998).

In dem folgenden Versuch wurde mit Hilfe des ELISA-Verfahrens die Konzentration an 14-3-3 Protein in verschiedenen Rinder-Serumproben (BSE-erkrankte Rinder und nicht-infizierte Kontrollen) sowie im Liquor cerebrospinalis (Patienten mit Creutzfeldt Jakob-Erkrankung) bestimmt.

### Durchführung:

1. Maleimid-aktivierte Mikrotiterplatte (Reacti-Bind® von der Firma Pierce; Cat-No. 15150) mit 100 µl Peptid-Lösung {100 µg/ml CAALPKINRSApSEPSLHR und 60 µM TCEPHCI [Tris-(2-carboxyethyl)-phosphin HCl ; Firma Pierce] in PBS/1 mM EDTA (pH 7,0 ; PBS = Phosphatpuffer-Salz-Lösung, bestehend aus 1,15 mM KH₀PO₄, 8,1 mM Na₂HPO₄, 137 mM NaCl und 2.7 mM KCI)} pro Well über Nacht bei Raumtemperatur unter leichtem Schütteln inkubieren.
2. 4-mal mit 200 µl PBS (pH 7.4) pro Well waschen.
3. Blockierung freier Maleimidgruppen auf der Platte durch 3-stündige Inkubation bei Raumtemperatur mit 200 µl 3% Rinderserumalbumin (BSA), 10 µg/ml Cystein und 120 µM TCEPHCI in PBS/1 mM EDTA (pH 7,0).
4. Platte ausschütteln und 4-mal mit 200 µl PBS (pH 7,4) pro Well waschen.
5. Inkubation mit 100 µl Probenlösung (Liquor cerebrospinalis oder Liquorverdünnung bzw. Serum oder Serumverdünnung) pro Well für 1 Stunde bei Raumtemperatur.
6. 4-mal mit PBS waschen (wie oben).
7. Zugabe von Anti-14-3-3 Protein-Antikörper (anti-14-3-3y, cat. no. sc-731, Firma Santa Cruz Biotechnology; Verdünnung 1 : 2000) in PBS (pH 7,4, enthaltend 3% BSA) und Inkubation für 2 Stunden bei Raumtemperatur auf Schüttler; anschließend 4-mal mit PBS waschen (wie oben).
8. Zugabe von 150 µl Peroxidase-konjugiertem Anti-Kaninchen-lmmunoglobulin (Verdünnung 1 : 2000) in PBS (pH 7,4, enthaltend 3% BSA) pro Well und Inkubation für 1 Stunde bei Raumtemperatur ; anschließend 4-mal mit PBS waschen (wie oben).
9. Zugabe von 100 µl Substratpuffer pro Well [Substratpuffer : 1 ml einer 0,4% igen ortho-Phenylendiamin (OPD)-Lösung plus 9 ml Substratpuffer plus 10 µl H₂O₂].
10. Nach etwa 10 Minuten Messung der optischen Dichte bei 490 nm in einem ELISA-Reader.

### Kontrollen :

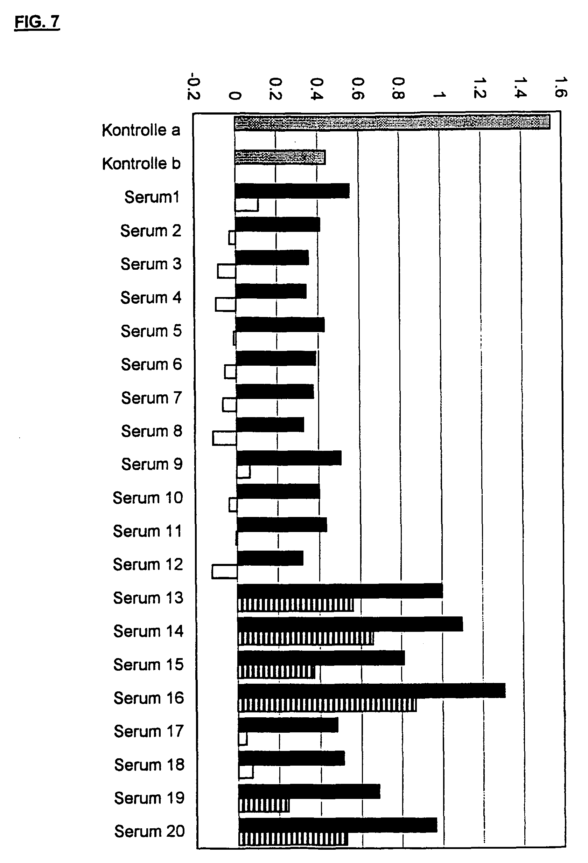

a) ohne Peptid und ohne Blockierungspuffer mit beiden Antikörpern
b) mit Peptid mit BSA-Blockierung mit beiden Antikörpern ABB. 7 zeigt, daß die mittels des ELISA-Verfahrens bestimmten Konzentrationen an 14-3-3 Protein in den Rinder-Serumproben 13,14,15,16,19 und 20, nicht jedoch bei den Kontrollen erhöht sind.

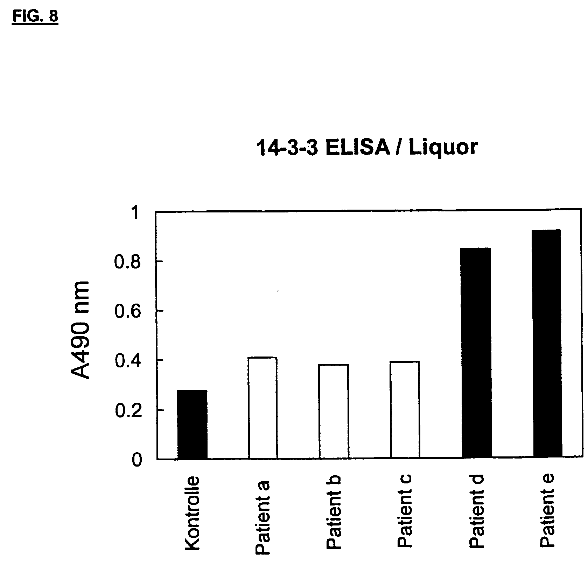

Mit Hilfe des ELISA-Verfahrens läßt sich auch die erhöhte Konzentration an 14-3-3 Protein in Liquor cerebrospinalis (Cerebrospinalflüssigkeit)-Proben von Patienten mit Creutzfeldt-Jakob-Erkrankung nachweisen. Wie ABB. 8 zeigt, liegt der 14-3-3 Protein-Spiegel in Liquor cerebrospinalis von zwei Patienten mit Creutzfeldt-Jakob Erkrankung (Patient d und e) deutlich höher als derjenige von gesunden Kontrollpersonen (Patient a-c).

### Beispiel 5: Quantifizierung von 14-3-3 Protein in Rinderhirn: Bestimmung der Empfindlichkeit des ELISA-Verfahrens

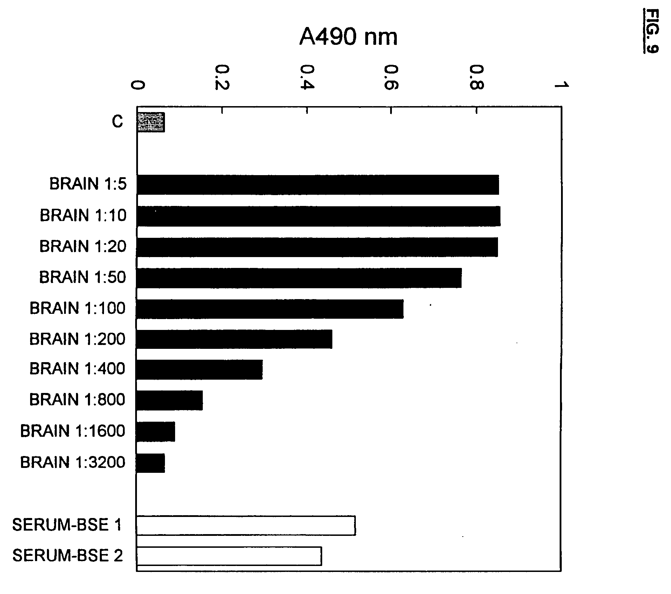

Zur Bestimmung der Empfindlichkeit der ELISA-Methode wurde eine Verdünnungsreihe von Rinderhirn-Extrakt mit einer bekannten Konzentration an 143-3 Protein hergestellt und darin 14-3-3 Protein mit Hilfe der ELISA Methode gemessen. Wie ABB. 9 zeigt, liegt die Nachweisgrenze von 14-3-3 Protein bei etwa 1 ng (Probenvolumen: 100 µl), also bei einer Konzentration von etwa 10 ng/ml an 14-3-3 Protein. Die Nachweisgrenze kann noch weiter herabgesetzt werden, beispielsweise durch späteres Abstoppen der Farbreaktion auf unter 1 ng/ml. Die in Seren (ABB. 9) bzw. Liquor (nicht gezeigt in ABB. 9) von BSE-infizierten Rindern vorkommenden Konzentrationen an 14-3-3 Protein liegen innerhalb des Meßbereichs des ELISA-Verfahrens.

Die Nachweisgrenze der konventionellen Western-Blotting-Methode liegt dagegen bei etwa 40 ng/ml; diese Methode ist also viel weniger empfindlich als das von uns entwickelte ELISA-Verfahren.

### 3. Erläuterungen zu den Abbildungen

Im nachfolgenden sind die erläuternden Legenden zu den auf den Seiten 1/9-9/9 abgebildeten Grafiken aufgeführt:
ABB. 1 zeigt eine schematische Darstellung der Rolle von 14-3-3, einem "lenkenden" Chaperon, und HSP70, einem Protein-Faltungs-Chaperon. Im Text ist ausgeführt, daß durch PCB 118 eine Induktion des Gens für 14-3-3-untersucht an dem Meeresschwamm *Geodia cydonium* stattfindet. Nach Dimerisierung bindet 14-3-3 an das Bindungsmotiv, RSxSxP [nach erfolgter Phosphorylierung] von Schlüsselmolekülen, die in intrazelluläre Signaltransduktionskaskaden oder in den Apoptose-Prozess involviert sind und verhindert deren Translokation in dasjenige Zellkompartiment, in dem die betreffende Funktion - wie Initiation einer Genexpression oder auch Apoptose - eingeleitet werden soll. Im Gegensatz dazu bindet HSP70 an Zielproteine und ermöglicht deren funktionelle Faltungen.

ABB. 2 zeigt einen Vergleich des abgeleiteten 14-3-3 Proteins von G. *cydonium* [GEODIAge] mit folgenden Isoformen: γ der Ratte [RAT-gamma], η vom Mensch [HOMO-eta], ξ vom Schaf [SHEEP-zeta], dem 14-3-3-verwandten Polypeptid von *Xenopus laevis* [XENLA-D2,214097], β der Ratte [RAT-beta], θ der Ratte [RATtheta], *Drosophila melanogaster* 14-3-3 Protein [DROME-LP], dem 14-3-3 Protein von *Caenorhabditis elegans* [CAEL-cds4] und σ vom Mensch [HOMO-sigma]. Konservierte Aminosäuren in allen Sequenzen sind invertiert dargestellt; solche die in mehr als fünf Sequenzen vorkommen, sind schattiert unterlegt. Die Sequenz, die zur Antikörperproduktion diente, ist gekennzeichnet (*****).

ABB. 3 zeigt den Effekt von PCB 118 auf die Expression der mRNA von 14-3-3 nach Inkubation der Tiere mit 2 µg PCB 118/g Feuchtgewicht für 0,5 bis 6 Tage. Die Untersuchungen wurden mittels Northern-Blotting [14-3-3-Sonde] (A) bzw. Western Blotting [14-3-3-Antikörper] (B) durchgeführt.

In ABB. 4 ist die Strukturformel von PCB 118 dargestellt.

In ABB. 5 ist der Effekt von PCB 118 (2 µg/g) und 17ss-Östradiol (20 ng/g) auf die Expression des 14-3-3 Gens gezeigt. Die beiden Chemikalien wurden entweder alleine oder in Kombination den Versuchstieren (*G. cydonium*) zugesetzt. Die Inkubationszeit wurde auf 3 bzw. 6 Tage begrenzt.

ABB. 6 zeigt eine schematische Darstellung des Prinzips des entwickelten ELISA Verfahrens zur quantitativen Bestimmung von 14-3-3 (Variante 2).

ABB. 7 zeigt die Konzentration an 14-3-3 Protein in Serumproben von Rindern (angegeben ist auf der y-Achse die Extinktion, die der 14-3-3 Protein-Konzentration proportional ist). Kontrollen (graue Balken): (a) ohne Peptid und ohne Blockierungspuffer mit beiden Antikörpern; (b) mit Peptid und mit BSA-Blockierung und mit beiden Antikörpern. Die schwarzen Balken zeigen die Meßwerte, die weißen und quergestreiften Balken die Meßwerte nach Subtraktion der Kontrolle b. Durchgeführt wurden jeweils Doppelbestimmungen. Die Seren 13,14,15,16,19 und 20 zeigen einen erhöhten 14-3-3 Protein-Spiegel (quergestreifte Balken).

ABB. 8 zeigt die Konzentration an 14-3-3 Protein in 5 humanen Liquor cerebrospinalis-Proben (angegeben ist auf der y-Achse die Extinktion, die der 14-33 Protein-Konzentration proportional ist). Die Proben d und e (schwarze Balken) sind von Patienten mit Creutzfeldt-Jakob-Erkrankung, die Proben a-c (weiße Balken) sind von Gesunden. Der graue Balken ist die Negativkontrolle.

ABB. 9 zeigt die Meßwerte für die Konzentration an 14-3-3 Protein in einer Verdünnungsreihe von Rinderhirn-Extrakt (schwarze Balken ; angegeben ist auf der y-Achse die Extinktion, die der 14-3-3 Protein-Konzentration proportional ist). Die angegebenen Verdünnungsstufen entsprechen folgenden Mengen an 14-3-3 Protein : 280 ng (Verdünnungsstufe 1 : 5), 140 ng (Verdünnungsstufe 1 : 10), 70 ng (Verdünnungsstufe 1 : 20), 28 ng (Verdünnungsstufe 1 : 50), 14 ng (Verdünnungsstufe 1 : 100), 7 ng (Verdünnungsstufe 1 : 200), 3.5 ng (Verdünnungsstufe 1 : 400), 1.75 ng (Verdünnungsstufe 1 : 800), 0.88 ng (Verdünnungsstufe 1 : 1600) und 0.44 ng (Verdünnungsstufe 1 : 3200). Zum Vergleich sind die Werte der Serumproben von zwei BSE-infizierten Rindern gezeigt (weiße Balken). Negativkontrolle (grauer Balken).

## Patentansprüche

1. Verfahren zum Aufspüren von natürlichen und anthropogenen Umweltchemikalien, insbesondere von polychlorierten Biphenylen und/oder (Xeno) Östrogenen in Gewässern oder im Boden, **dadurch gekennzeichnet, daß** 14-3-3 Proteine als Biomarker in Bioindikatororganismen (Vertebraten und Invertebraten), die dem zu untersuchenden Gewässer oder Boden ausgesetzt werden, bestimmt werden.

2. Verfahren zum Nachweis und zur Bestimmung der Konzentration von 14-3-3 Protein(en) (= ELISA-Verfahren), das **dadurch gekennzeichnet ist, daß** zur spezifischen Bindung (und Konzentrierung) der 14-3-3 Proteine Mikrotiterplatten verwendet werden, die mit einem Peptid, welches ein Bindemotiv des 14-3-3 Proteins besitzt, beschichtet sind. Beispielsweise können Maleimid-aktivierte Mikrotiterplatten verwendet werden, an die das chemisch synthetisiertes Peptid CAALPKINRSApSEPSLHR (pS Phosphoserin), welches mit hoher Affinität an die 14-3-3 Proteine bindet, durch Reaktion der Sulfhydrylgruppe des N-terminalen Cysteins gebunden wird. Nach Zugabe der zu untersuchenden Extrakte oder Körperflüssigkeiten in die Wells der Mikrotiterplatten erfolgt die Detektion und Quantifizierung der gebildeten Peptid-14-3-3 Protein-Komplexe zum Beispiel über markierte Antikörper wie alkalische Phosphatase-konjugierte Antikörper. Alternativ können Streptavidin beschichtete Mikrotiterplatten verwendet werden, an die ein biotinyliertes Peptid mit dem Bindemotiv des 14-3-3 Proteins bindet ; die Inkubation des biotinylierten Peptids mit der Probe (Bildung der Peptid-14-3-3 Protein Komplexe) wird entweder vor oder nach dessen Bindung an die Platte durchgeführt. Die Nachweisgrenze des ELISA liegt bei mindestens 10 ng 14-3-3 Protein/ml und kann, beispielsweise durch späteres Abstoppen der Farbreaktion, auf unter 1 ng/ml gesteigert werden.

3. Verwendung des in Anspruch 2 genannten ELISA-Verfahrens zum Nachweis und zur Quantifizierung von 14-3-3 Protein in Vertebraten und Invertebraten, die natürlichen und anthropogenen Umweltchemikalien, insbesondere von polychlorierten Biphenylen und/oder (Xeno) Östrogenen ausgesetzt werden.

4. Verwendung des in Anspruch 2 genannten ELISA-Verfahrens zum Nachweis und zur Quantifizierung von 14-3-3 Protein in Körperflüssigkeiten (beispielsweise Cerebrospinalflüssigkeit und Serum) von Menschen und Tieren, die mit dem Erreger von Prion-Erkrankungen infiziert sind.
